# EUROPEAN PATENT SPECIFICATION

(11) **EP 3 244 783 B1**
(45) Date of publication and mention of the grant of the patent: **17.10.2018**
(21) Application number: 16715351.9
(22) Date of filing: 08.01.2016
(51) Int. Cl.: A47K 7/04, A47K 10/48, A61B 90/80

(54) **WASHING DEVICE FOR WASHING OBJECTS AND METHOD THEREFOR**
WASCHVORRICHTUNG ZUM WASCHEN VON GEGENSTÄNDEN UND VERFAHREN DAFÜR
DISPOSITIF DE LAVAGE POUR LE LAVAGE D'OBJETS ET PROCÉDÉ ASSOCIÉ

(30) Priority: 13.01.2015 NL 2014125
(43) Date of publication of application: 22.11.2017
(73) Proprietor: Sita Holding B.V., 1213 XX Hilversum (NL)
(72) Inventor: MELEIN, Jan Baptist Franciscus, 9003 MC Warten (NL); MELEIN, Ane Andries Pieter, 9728 XR Groningen (NL)
(74) Representative: Verdijck, Gerardus
(86) International application number: PCT/NL2016/050012
(87) International publication number: WO 2016/114650

(56) References cited:
- WO-A1-2012/030216
- JP-A- H1 132 941
- JP-A- 2000 262 433
- KR-B1- 101 231 844
- US-A1- 2012 260 418

## Description

The invention relates to a washing device for washing objects, in particular for washing hands.

Conventional washing devices are usually provided with a tap. This has the drawback that a relatively large amount of water is used to wash for instance hands, on average about 1 litre per washing session. In the case the washing device has a hot water tap, energy is moreover consumed in order to heat the quantity of water used.

Known from WO 2012/030216 is a washing device in which water is atomized for the purpose of washing hands. This reduces the quantity of water necessary per washing session. WO 2012/030216 describes the use of insulated liquid storage for maintaining a quantity of water at a temperature of at least 80°C. Maintaining a considerable quantity of water continuously at such a relatively high temperature does however require energy.

KR 101 231 844 B1 discloses a hand washing apparatus wherein hands can be dried and washed, according to the preamble of claim 1.

An object of the invention is to reduce the above stated drawbacks of the known washing devices and to provide a washing device which consumes a small quantity of water per washing session and which is moreover energy-efficient.

The object is achieved with the washing device for washing objects such as hands according to claim 1.

By providing an atomizer it is possible to suffice with a small quantity per washing session, while objects still become just as clean as with conventional washing devices. The sprayer is for instance provided in a wall of the washbasin.

In a first embodiment the heater comprises a body of a heat-conductive material, wherein a heating element for heating the body and a channel for carrying water for heating through the body are arranged in the body.

The body is heated with the heating element. As soon as the water is carried through the channel in the body, the heated body will heat the water. In conventional washing devices a considerable quantity of water is continuously held at temperature. Use is usually made in such devices of water as storage liquid for the heat. Due to the presence of an atomizer however, the washing device according to the invention uses a small quantity of water per washing session. Holding a large quantity of water at temperature by means of water as storage means as in conventional devices is therefore not energy-efficient. It is however possible to bring small quantities of water to temperature quickly using the heater according to the invention.

A relatively small quantity of water can moreover be held at temperature according to the invention, wherein the channel in the body functions as storage for hot water. A dripper or valve is for instance provided at the exit of the channel. The dripper is switched on or the valve opened, for instance by a controller, as soon as heated water has to be carried to the sprayers. The dripper is switched off or the valve closed when there is no demand, whereby the water in the channel is held at temperature.

The heating element can be integrated into the body of the heater, for instance by casting the heating element in the body. In another embodiment the heating element is inserted into a close-fitting opening of the heater body. In a third embodiment the heating element is placed in an opening in the heater body, which opening is subsequently filled with a heat-conductive material, for instance a heat-conductive paste.

The body is preferably embodied as a solid body.

The heat-conductive material for instance comprises a metal, which in the context of the invention is also understood to mean a metal alloy. The heat-conductive material preferably comprises aluminium, for instance in the form of an aluminium alloy.

The heating element is preferably separated from the channel. That is, the channel preferably makes no contact with the heating element, so that in use water flowing through the channel is not in direct contact with the heating element. In that case the heating element heats the body, and the heated body heats the water flowing through the channel in the body.

The inner wall of the channel can be provided with a coating, for instance an anti-corrosion coating and/or a plastic coating.

A further advantage of the heater according to the invention is that it is easy to produce compared to conventional boilers.

The washing device for instance comprises a controller connected to the heating element for the purpose of holding the body at a temperature within a predetermined temperature range. The temperature of the body is preferably held below the boiling point of water at the given pressure. For atmospheric pressure the body is for instance held at temperature of a minimum of 50 degrees, preferably at a temperature of a minimum of 60 degrees Celsius, more preferably at a temperature of a minimum of 70 degrees Celsius and most preferably at a temperature of 80-95 degrees.

In a preferred embodiment the channel has a volume of less than 400 ml, preferably less than 200 ml, more preferably less than 100 ml and most preferably a maximum of 50 ml.

The washing device is preferably configured to spray per washing session about 5-100 ml, more preferably 5-50 ml, still more preferably 5-25 ml and most preferably 5-10 ml of water.

The body preferably has a volume of at least 5 times, more preferably at least 8 times, still more preferably at least 10 times, and most preferably at least 12 times the volume of the channel.

The body for instance has a volume of 600 cm³ and the channel a volume of 50 ml.

In a preferred embodiment the washing device comprises a dripper. The dripper is for instance provided between the water inlet and the heater or between the heater and the sprayer. It is likewise possible to provide a plurality of drippers, for instance between the water inlet and the heater as well as between the heater and the sprayer.

In a further preferred embodiment the dripper is configured to supply water at a flow rate of less than 20 ml/s, preferably less than 10 ml/s, more preferably less than 5 ml/s and most preferably a maximum of 2 ml/s.

According to the invention, the device comprises an air feed conduit preferably comprising a pump and/or an air filter.

The air feed is applied to atomize the water, therefor the air feed is connected to the atomizer. The air is preferably supplied under pressure via the air feed conduit, for instance by means of a pump or other compressed air installation.

Alternatively or additionally the air feed is preferably applied to dry the washed object at the end of the washing cycle.

The air is supplied via the air feed conduit. A pump and/or an air filter are provided in the feed conduit. The pump can be provided upstream or downstream of the air filter in the flow direction. Achieved by means of the air filter is that washing, and the optional drying, take place hygienically.

In a further preferred embodiment the washbasin further comprises an extraction opening which is preferably connected via an air recirculation conduit to the pump for recirculation of air.

Particularly when use is made of air pressure for atomizing it is advantageous to provide an extractor to prevent an overpressure in the washbasin, and thereby splashing of water. It has been found particularly advantageous to extract the air by means of the same pump used to supply air. The extraction opening is for instance provided in a lower zone of the washbasin, while the atomizer is provided in an upper zone of the washbasin. At least one extraction opening is preferably provided in an upper zone of the washbasin, for instance above the atomizer.

The recirculated air is filtered in that a filter is also provided as well as a pump. Bacteria and dirt possibly present in the extracted air are hereby filtered out of the air before it is carried to the atomizer.

The air filter preferably comprises a substantially cylindrical air chamber, wherein an air inlet is provided in the curved side wall of the air chamber, which air inlet is configured to form a circular flow in the air chamber and wherein an air outlet is provided in the upper or lower side of the air chamber, which air outlet has a diameter smaller than the diameter of the air chamber and is provided close to the longitudinal axis of the air chamber, wherein the air filter comprises a UV light source configured to shine UV light into the air chamber.

The air filter is embodied for instance as a cyclone. In addition to having an air outlet, a cyclone usually also has an outlet for solid particles. According to the invention an outlet for solid particles is however optional and the air filter preferably does not comprise such an outlet, although in an alternative embodiment an outlet can be provided if desired.

The air inlet is preferably provided tangentially on the cylindrical air chamber so as to thus bring about a circular, or at least substantially circular flow of the air.

Micro-organisms are urged by the air filter in the direction of the inner wall of the air chamber where they continue to circulate for a longer period of time. Since the air outlet is placed close to the longitudinal axis of the cylindrical air chamber, i.e. close to the centre of the air chamber, heavier particles such as micro-organisms remain in the air chamber for longer. Micro-organisms which may be present are hereby exposed to the UV light for a relatively long period of time. This provides for a more effective killing of micro-organisms than in conventional air filters.

In a further preferred embodiment the inner wall of the air chamber is provided with titanium dioxide. UV light in combination with titanium dioxide provides for the forming of free radicals. These free radicals are introduced via the pump into the washbasin, this resulting in killing of bacteria through the whole device, at least through all parts of the device through which the air with free radicals flows. The free radicals moreover provide for disintegration of organic materials. The device hereby has a certain self-cleaning capability. This enhances washing hygiene.

In a preferred embodiment the pump is accommodated in a heat-insulated housing and is switchable by means of a thermostat between a mode of use, in which the pump carries air via the air feed conduit to the atomizer, and a cooling mode in which the pump draws in outside air.

The pump will heat up during use. This has the advantage that heated air is supplied for the purpose of for instance drying objects at the end of the washing cycle. The temperature of the air must however not become so warm that this is unpleasant for the user or that the pump becomes overheated. The pump temperature is therefore controlled by means of a thermostat, wherein the pump draws in outside air when the temperature of the pump rises too high.

In a further preferred embodiment the body of the heater is accommodated with the pump in the heat-insulated housing.

This has the advantage that the heat of the pump is used to hold the body of the heater at temperature, and vice versa. The washing device according to the invention is hereby energy-efficient. The heating element need in particular be switched on less often to heat the body, or the pump need operate for less long a time in order to reach the desired temperature.

In an unclaimed embodiment the above embodiments including the pump can be provided in combination with a heater other than stated above. Such a washing device comprises:
- a washbasin provided with an outlet;
- an inlet for supplying water;
- a heater connected to the inlet and configured to heat water supplied from the inlet; and
- a sprayer connected to the heater and configured to spray heated water supplied from the heater into the washbasin, which sprayer comprises an atomizer for forming a spray of water droplets in the washbasin,
further comprising an air feed conduit comprising a pump and an air filter. The air filter is preferably an air filter with a UV light source as described above, wherein titanium dioxide is more preferably provided on the inner wall of the air chamber.

In the first and/or second embodiment the sprayer preferably comprises an outlet opening wherein an extraction channel connected to an extractor is provided all around the outlet opening.

The extraction channel around the outlet opening of the sprayer prevents accumulation of water around the outlet opening. When a drying phase is started after water has been sprayed onto the object for washing, these accumulated droplets can be entrained in the drying airflow. The object hereby becomes moist again, which makes the drying more difficult. Providing an extraction channel around the sprayers therefore achieves that an efficient drying process can take place at a later stage.

The extraction channel is for instance connected to the suction side of the pump which serves for supply of air to the atomizer.

In a further embodiment the device further comprises a device for deionization of water, for instance a device for capacitive deionization of water. Additionally or alternatively the washing device comprises another device for pretreating water. The pretreatment comprises for instance deionization or demineralization. The washing device for instance comprises a device for reverse osmosis for demineralization, or a device for treating water with radio waves and/or magnetic fields.

Deionization and/or demineralization of water prevents mineral deposition such as limescale formation. Since it is possible to operate with small volumes of water, mineral deposition can easily block the relatively small conduit system. Limescale deposits in particular can moreover have an adverse effect on the heater. These problems are countered by means of a deionization device and/or a demineralization device. The deionization device and/or demineralization device is for instance connected downstream of the water inlet.

The invention also relates to a method for washing objects, comprising of providing a washing device as described above. The same advantages and effects as described above in relation to the washing device according to the invention also apply to the method according to the invention.

Further advantages, features and details of the invention are elucidated on the basis of preferred embodiments thereof, wherein reference is made to the accompanying drawings.
- Figure 1 shows schematically an exemplary embodiment of a washing device according to the invention, in particular for washing hands;
- Figure 2 shows a cross-section of a part of the washing device of figure 1;
- Figure 3 shows a block diagram of several components of the washing device, and furthermore the air and water flows between these components;
- Figure 4 shows schematically an example of a heater according to the invention provided with a thermostat; and
- Figure 5 shows schematically an air filter for a washing device according to the invention.

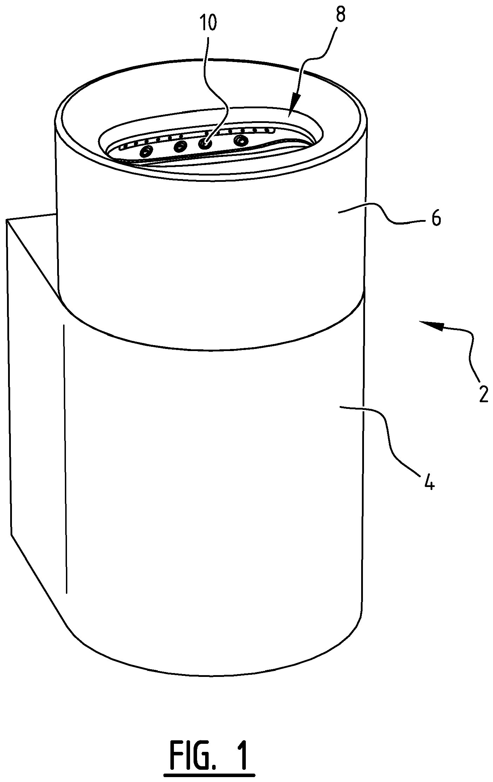

Washing device 2 (figure 1) comprises a housing 4 on which a washbasin 6 is provided. Washbasin 6 is provided on its upper side with an opening 8. In an upper part of washbasin 6 a number of sprayers 10 are provided in the wall. Provided around the sprayers 10 is a channel 11 which is connected to an extractor, i.e. an extraction channel 11 is provided all around each of the sprayers 10.

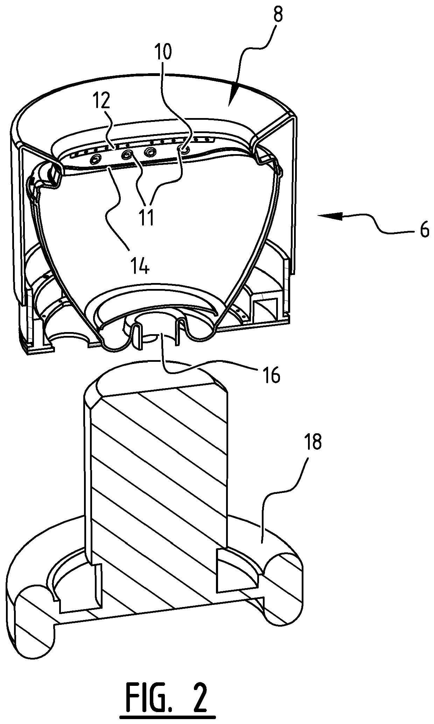

The upper part of washbasin 6 is optionally provided with lighting 12 (figure 2), in this example in the form of LED lighting. Optionally also provided in the washbasin is an air outlet 14 for drying hands. An outlet 16 via which water can be drained is provided on the underside of washbasin 6. Figure 2 moreover shows schematically a pump 18 for extracting air from washbasin 6 and/or supplying air to the atomizer and/or outlet openings 14.

An extractor is preferably also provided above sprayers 10, below the edge of washbasin 6.

Washbasin 6 optionally comprises a sensor (not shown) for detecting one or more hands in washbasin 6. The sensor is for instance a proximity sensor, such as an infrared sensor. In another embodiment the sensor is embodied as a light barrier, for instance an infrared light barrier. The washing cycle is preferably started automatically upon detection of hands in the washbasin. The washing cycle can alternatively be started manually by pressing a knob which can for instance be operated by means of the foot of a user.

According to the invention it is optionally also possible for an additional motion sensor ("outer sensor") to detect when someone is close to the hand washer. LED lighting 12 for instance comes on on the basis of this detection. LED lighting 12 switches off for instance when no hands have been placed into washbasin 6 within a predetermined time, for instance 10 seconds.

During the washing cycle the hands are cleaned with water in fast flowing air during a first step which for instance lasts for 5 seconds. In a second step, which for instance lasts for 10 to 15 seconds, the hands are then dried by the airflow without water. This drying time can optionally be increased by holding the hands longer in the basin.

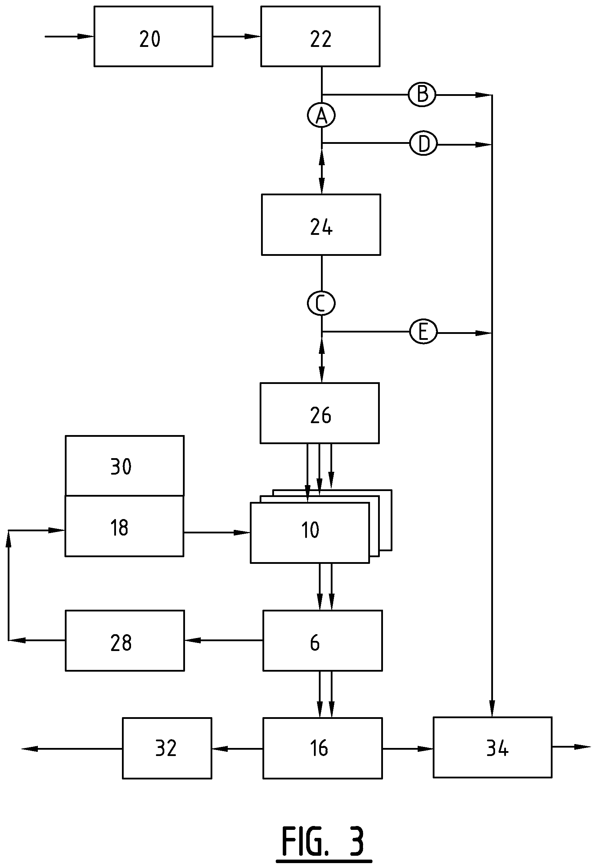

The washing cycle is controlled in the shown embodiment by switching on the air pump, operating a number of valves and subsequently switching off the air pump. The washing cycle is elucidated below with reference to figure 3. Four valves are applied in this embodiment.

The washing device comprises water tap 20, softener 22, which in the shown embodiment is embodied as a device for capacitive deionization, heater 24, a distributor/mixer 26, sprayers 10, washbasin 6, air filter 28, air pump 18, inverter 30, receptacle 16, fan 32 and water outlet 34. Valves A, B, C and E are electric valves of the "normally closed" type. Valve D is a pressure relief valve. Valve A can alternatively be embodied as non-return valve.

At the start of the washing cycle valves A and C are opened and water is carried in the first step from heater 24 to distributor 26. Water from softener 22 replenishes the water in heater 24 and water from water tap 20 enters softener 22 for softening.

The water is distributed by distributor 26 over a number of hoses and transported via the hoses to sprayers 10. Here the water is injected into an airflow and separated into a large number of fine droplets. The air has for instance a speed of 100-200 m/s, preferably about 150 m/s.

The hoses are provided with drippers in order to control the throughflow speed. A dripper is for instance provided in the connection between water tap 20 and softener 22 and/or the connection between softener 22 and heater 24 and/or the connection between heater 24 and distributor 26 and/or the connection between distributor 26 and sprayers 10. The flow rate of the water through the drippers is for instance limited to 2 ml/s. Additionally or alternatively to the drippers can be provided a pump, for instance with the same flow rate as the dripper, for instance of about 2 ml/s.

In washbasin 6 the droplets are blown against the hands and there clean the hands. The water droplets are subsequently collected in receptacle 16, where the droplets are separated from the airflow and disappear under the influence of gravitational force into outlet 34.

LEDs 12 optionally illuminate the water spray, whereby the user can see that the washing cycle is active and his/her hands are being cleaned. At the end of the first step valves A and C close so that the water supply to washbasin 6 stops. In the second step of the washing cycle the air supply to sprayers 10 remains activated, although because valves A and C are closed water is no longer sprayed. Valve E is opened so that, because of the prevailing air pressure, residual water from distributor 26 goes to water outlet 34. Only warm air now comes from sprayer holes 10, which blows the hands dry.

LEDs 12 optionally begin to flash in order to mark the transition to the drying step. The drying step will in any case last a predetermined drying time, for instance 10 seconds. This is for instance indicated by means of LEDs 12 which, for instance at the end of the predetermined drying time, display a different colour or go out or begin to flash. Following the predetermined drying time the supply of warm air to sprayers 10 remains active as long as the sensor detects hands in washbasin 6. The additional drying time is optionally limited, for instance to 5 seconds. When the user takes his/her hands out of the basin, or the maximum drying time has been reached, the motor of pump 18 runs down and the user can take his/her hands out of the basin if he/she has not already done so.

As soon as the hands have been taken out of washbasin 6 the hand washer is ready for the following user. This is also possible when the motor of pump 18 is running down. If hands are already detected again during running down, the motor will restart.

After a predetermined number of washing cycles, for instance 10-100 washing cycles, the polarity on softener 22 is reversed. Valve B is then opened while valve A remains closed. The content of softener 22 hereby flows to water outlet 34 and is replenished with tap water. The softener 22 is hereby flushed clean. As soon as softener 22 is clean enough, or as soon as a new washing cycle is started, valve B closes and the polarity of the softener is switched back again so that softener 22 is once again ready for use. In the case the flushing stops prematurely, another attempt is made after the next washing cycle.

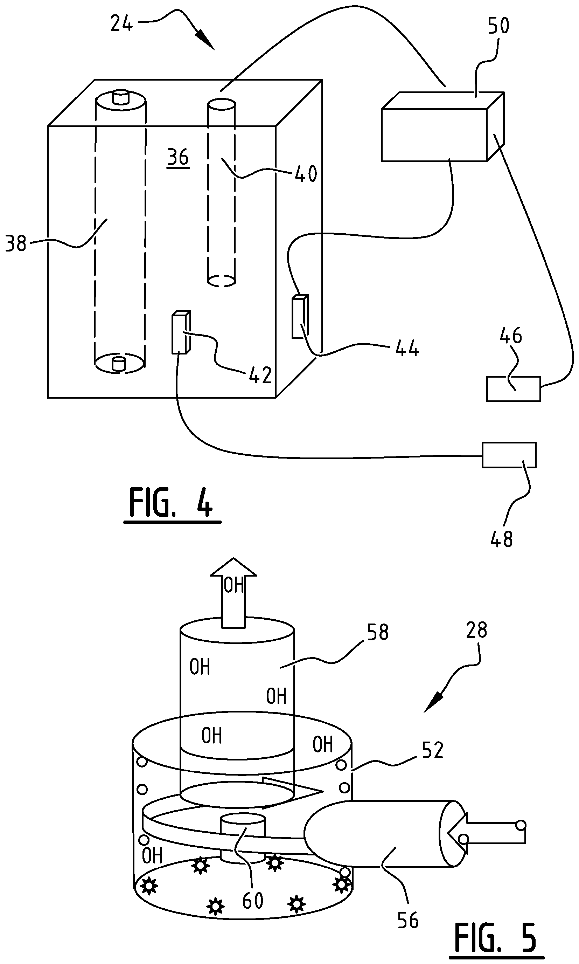

Heater 24 (figure 4) comprises a block of aluminium 36. A continuous channel 38 for water is arranged in block 36. In addition, a recess is arranged in block 36 in which a heating element 40 is placed. The recess is subsequently filled with a heat-conductive paste. Channel 38 is closed at its outer ends with a hose coupling for connection to feed and discharge hoses. On the discharge side, and optionally also the feed side, use is made of a heat-resistant hose and a heat-resistant hose coupling.

Sensors 42, 44 monitor the temperature of block 36. Heating element 40 is powered by a power supply 46, in this embodiment a 230 V voltage source. Connected between power supply 46 and heating element 40 is a thermostat switch 50 which is also connected to sensor 44. On the basis of the temperature detected by sensor 44 thermostat 50 switches heating element 40 on or off so that the temperature is held at a predetermined value. Block 36 is preferably held at a temperature of about 95 degrees Celsius.

Optional sensor 42 is connected to the controller of the washing device. As additional safety measure, the controller switches the heater off at a temperature above 100 degrees Celsius.

During use cold water flows into the heated block 36 via channel 38. The water is heated by the heat of block 36 which is held at a temperature of about 95 degrees Celsius. Because it relinquishes heat, block 36 will cool. If the temperature of block 36 falls below a preset threshold value, for instance 90 degrees Celsius, the thermostat switch then switches on electrical heating element 40. The temperature of block 36 then increases again.

If the temperature rises too high, the water could begin to boil, thereby resulting in an overpressure. Water is then carried in the direction of outlet 34 by pressure relief valve D (figure 3). The washing device moreover switches off heating element 40 via the thermostat or an overall controller.

Block 36 can be provided on its outer side with insulating material (not shown in the figure). Block 36 is however preferably accommodated together with pump 18 in an insulated housing. The housing is for instance insulated by a layer of rockwool and/or an insulating foil.

Pump 18 is held at a temperature of 30-50 degrees Celsius in the housing. The air displaced by pump 18 is in this way heated to about this temperature. The heated air is for instance used to dry hands.

A controller monitors the temperature of pump 18. If the temperature rises above a determined threshold value, the controller switches pump 18 to a cooling mode in which cold outside air is drawn in. Pump 18 preferably operates at moderate speed in the cooling mode.

In the shown embodiment the rotation direction of pump 18 is reversed in the cooling mode using inverter 30. Because of a non-return valve in the supply conduit to sprayers 10 (not shown in figure 3) the pump now draws in outside air and the compression is moreover released. The pump hereby cools.

If the temperature rises above a second upper limit, for instance 60 degrees Celsius, the controller then switches the washing device to a failure mode, in which no further washing program can be performed, in order to prevent damage to the device. The washing device is completely disabled in the failure mode, with the exception of an optional failure indicator.

When the washing device has not been used for some time, the temperature of the pump will fall. When the pump temperature falls below a lowest threshold value, for instance 35 degrees, the controller then switches on pump 18 so that it heats up. The pump can be held continuously at the desired temperature. The pump is alternatively brought to temperature as soon as a user is detected in the vicinity of the device or if hands are detected in washbasin 6.

A second lower limit for the temperature is moreover optionally defined in the controller. If the temperature falls below the second lower limit of for instance 0-5 degrees Celsius, the controller switches the washing device to the failure mode in order to prevent damage to the device.

The airflows of the washing device will be elucidated hereinbelow with reference to figure 3. Air is drawn in via a channel in the side wall of washbasin 6. This air is recirculated as blow air. The air drawn in from washbasin 6 is carried through an air filter 28 and subsequently guided via pump 18 in the direction of the sprayers. During the first step of the washing cycle the primary object of the supplied air is to atomize the water, during the second step of the washing cycle the air serves for the purpose of drying the washed hands. No further water is supplied to sprayers 10 in the second step of the washing cycle.

The air is disinfected by UV in filter 28. Free OH radicals are also introduced into the air by a photocatalytic reaction with titanium dioxide provided in the filter. Dirt particles, micro-organisms and odours are hereby rendered harmless everywhere in the device.

In addition, air is drawn in by a fan 32 which is connected to an outlet opening in a lower zone of washbasin 6. This air is filtered and provided with free OH radicals, and so is free of harmful micro-organisms.

Air filter 28 is further illustrated in figure 5. Filter 28 comprises a cylindrical air chamber 52 in which a UV light source 54 is provided. Air chamber 52 is moreover provided with an inlet 56 on its curved side wall and an outlet 58 on its upper side. In the shown embodiment outlet 58 has a smaller diameter than air chamber 52 and is moreover provided centrally on the upper surface of air chamber 52.

Air with possible microbiological contamination such as bacteria enters air chamber 52 at a certain speed via inlet 56. Inlet 56 connects asymmetrically to air chamber 52. As shown, inlet 56 is tangential to cylindrical air chamber 52. The introduced air begins to circulate as a result. Owing to the centrifugal action heavy particles move to the outer side of air chamber 52. These particles continue to circulate here for some considerable time, while a part of the air without heavy particles, or at least with fewer particles, is discharged via outlet 58.

Owing to the circular movement of the air the time for which the microbiological contamination such as bacteria remains in filter 28 is increased. The UV light from light source 54 therefore has more time to render them harmless. A large-scale elimination can still be obtained if desired with UV radiation of relatively low intensity and long wavelength (365 nm).

Titanium dioxide is provided on the inner wall of air chamber 52. This can for instance be provided as a titanium foil to which a layer of titanium dioxide is applied. Illuminating the titanium dioxide with the UV light from light source 54 results in a photocatalytic reaction in which OH radicals occur. These radicals are dispersed in the air through the whole device. These OH radicals are highly effective in eliminating micro-organisms. Organic material is moreover decomposed. The bacteria are hereby also killed elsewhere in the device and the formation of contamination on the wall is prevented.

Because the titanium dioxide is formed on titanium, for instance in the form of a foil, the titanium dioxide is wear-resistant.

The invention is by no means limited to the above described preferred embodiments thereof. The rights sought are defined by the following claims.

## Claims

1. Washing device (2) for washing objects such as hands, comprising:
- a washbasin (6) provided with an outlet (16);
- an inlet for supplying water;
- a heater (34) connected to the inlet and configured to heat water supplied from the inlet; and
- a sprayer (10) connected to the heater and configured to spray heated water supplied from the heater into the washbasin, ,
wherein the heater comprises a body (36) of a heat-conductive material, wherein a heating element (40) for heating the body and a channel (38) for carrying water for heating through the body are arranged in the body, **characterized in that** the sprayer comprises an atomizer for forming a spray of water droplets in the washbasin, and wherein the washing device comprises an air feed that is connected to the atomizer to atomize the water.

2. Washing device as claimed in claim 1, wherein the heat-conductive material comprises a metal, preferably aluminium.

3. Washing device as claimed in claim 1 or 2, wherein the channel has a volume of less than 400 ml, preferably less than 200 ml, more preferably less than 100 ml and most preferably a maximum of 50 ml.

4. Washing device as claimed in any of the foregoing claims, further comprising a dripper.

5. Washing device as claimed in claim 4, wherein the dripper is configured to supply water at a flow rate of less than 20 ml/s, preferably less than 10 ml/s, more preferably less than 5 ml/s and most preferably a maximum of 2 ml/s.

6. Washing device as claimed in any of the foregoing claims, comprising an air feed conduit comprising a pump (18) and an air filter (28).

7. Washing device as claimed in claim 6, wherein the washbasin further comprises an extraction opening which is connected via an air recirculation conduit to the pump for recirculation of air.

8. Washing device as claimed in claim 6 or 7, wherein the air filter (28) comprises a substantially cylindrical air chamber (52), wherein an air inlet (56) is provided in the curved side wall of the air chamber, which air inlet is configured to form a circular flow in the air chamber, and wherein an air outlet (58) is provided in the upper or lower side of the air chamber, which air outlet has a diameter smaller than the diameter of the air chamber and is provided close to the longitudinal axis of the air chamber, wherein the air filter comprises a UV light source (54) configured to shine UV light into the air chamber.

9. Washing device as claimed in claim 8, wherein the inner wall of the air chamber (52) is provided with titanium dioxide.

10. Washing device as claimed in any of the claims 6-9, wherein the pump is accommodated in a heat-insulated housing and is switchable by means of a thermostat (50) between a mode of use, in which the pump carries air via the air feed conduit to the atomizer, and a cooling mode in which the pump draws in outside air.

11. Washing device as claimed in claim 10, wherein the body of the heater (34) is accommodated with the pump in the heat-insulated housing.

12. Washing device as claimed in any of the foregoing claims, wherein the sprayer (10) comprises an outlet opening, wherein an extraction channel connected to an extractor is provided all around the outlet opening.

13. Washing device as claimed in any of the foregoing claims, further comprising a device for deionization of water, preferably a device for capacitive deionization of water.

14. Method for washing objects, comprising of providing a washing device as claimed in any of the claims 1-13.

## Patentansprüche

1. Waschvorrichtung (2) zum Waschen von Gegenständen, wie etwa Händen, die aufweist:
- ein Waschbecken (6), das mit einem Ablauf (16) versehen ist;
- einen Zulauf zum Zuführen von Wasser;
- eine Heizung (34), die mit dem Zulauf verbunden ist und aufgebaut ist, um von dem Zulauf geliefertes Wasser zu heizen; und
- einen Sprüher (10), der mit der Heizung verbunden ist und aufgebaut ist, um geheiztes Wasser, das von der Heizung geliefert wird, in das Waschbecken zu spritzen,
wobei die Heizung einen Körper (36) aus einem wärmeleitenden Material aufweist, wobei ein Heizelement (40) zum Heizen des Körpers und ein Kanal (38) zum Befördern von Wasser zum Heizen durch den Körper in dem Körper angeordnet sind, **dadurch gekennzeichnet, dass** der Sprüher einen Zerstäuber zum Ausbilden eines Spritzstrahls aus Wassertröpfchen in dem Waschbecken aufweist, und wobei die Waschvorrichtung eine Luftzuführung aufweist, die mit dem Zerstäuber verbunden ist, um das Wasser zu zerstäuben.

2. Waschvorrichtung nach Anspruch 1, wobei das wärmeleitende Material ein Metall, vorzugsweise Aluminium, aufweist.

3. Waschvorrichtung nach Anspruch 1 oder 2, wobei der Kanal ein Volumen von weniger als 400 ml, vorzugsweise weniger als 200 ml, bevorzugter weniger als 100 ml und am bevorzugtesten maximal 50 ml hat.

4. Waschvorrichtung nach einem der vorhergehenden Ansprüche, die ferner eine Tropfeinrichtung aufweist.

5. Waschvorrichtung nach Anspruch 4, wobei die Tropfeinrichtung aufgebaut ist, um Wasser mit einem Durchsatz von weniger als 20 ml/s, vorzugsweise weniger als 10 ml/s, bevorzugter weniger als 5 ml/s und am bevorzugtesten maximal 2 ml/s zu liefern.

6. Waschvorrichtung nach einem der vorhergehenden Ansprüche, die einen Luftzuführungskanal aufweist, der eine Pumpe (18) und ein Luftfilter (28) aufweist.

7. Waschvorrichtung nach Anspruch 6, wobei das Waschbecken ferner eine Entnahmeöffnung aufweist, die über einen Luftrezirkulationskanal mit der Pumpe für die Rezirkulation von Luft verbunden ist.

8. Waschvorrichtung nach Anspruch 6 oder 7, wobei das Luftfilter (28) eine im Wesentlichen zylindrische Luftkammer (52) aufweist, wobei ein Lufteinlass (56) in der gekrümmten Seitenwand der Luftkammer bereitgestellt ist, wobei der Lufteinlass aufgebaut ist, um eine kreisförmige Strömung in der Luftkammer zu bilden, und wobei ein Luftauslass (58) in der oberen oder unteren Seite der Luftkammer bereitgestellt ist, wobei der Luftauslass einen kleineren Durchmesser als den Durchmesser der Luftkammer hat und nahe der Längsachse der Luftkammer bereitgestellt ist, wobei das Luftfilter eine UV-Lichtquelle (54) aufweist, die aufgebaut ist, um UV-Licht in die Luftkammer zu strahlen.

9. Waschvorrichtung Anspruch 8, wobei die Innenwand der Luftkammer (52) mit Titanoxid versehen ist.

10. Waschvorrichtung nach einem der Ansprüche 8 - 9, wobei die Pumpe in einem wärmeisolierten Gehäuse aufgenommen ist und mittels eines Thermostaten (50) zwischen einer Verwendungsbetriebsart, in der die Pumpe über den Luftzuführungskanal Luft zu dem Zerstäuber befördert, und einer Kühlbetriebsart, in der die Pumpe Außenluft einsaugt, umschaltbar ist.

11. Waschvorrichtung nach Anspruch 10, wobei der Körper der Heizung (34) mit der Pumpe in dem wärmeisolierten Gehäuse aufgenommen ist.

12. Waschvorrichtung nach einem der vorhergehenden Ansprüche, wobei die Sprüheinrichtung (10) eine Auslassöffnung aufweist, wobei ein Entnahmekanal, der mit einer Entnahmeeinrichtung verbunden ist, ganz um die Auslassöffnung herum bereitgestellt ist.

13. Waschvorrichtung nach einem der vorhergehenden Ansprüche, die ferner eine Vorrichtung zur Entionisierung von Wasser, vorzugsweise eine Vorrichtung zur die kapazitive Entionisierung von Wasser, aufweist.

14. Verfahren zum Waschen von Gegenständen, das die Bereitstellung einer Waschvorrichtung nach einem der Ansprüche 1 - 13 aufweist.

## Revendications

1. Dispositif de lavage (2) pour le lavage d'objets tels que des mains, comprenant :
- un lavabo (6) doté d'une sortie (16) ;
- une entrée pour l'alimentation en eau ;
- un appareil de chauffage (34) raccordé à l'entrée et configuré pour chauffer l'eau alimentée par l'entrée ; et
- un pulvérisateur (10) raccordé à l'appareil de chauffage et configuré pour pulvériser de l'eau chauffée alimentée par l'appareil de chauffage dans le lavabo,
dans lequel l'appareil de chauffage comprend un corps (36) d'un matériau thermoconducteur, dans lequel un élément chauffant (40) pour chauffer le corps et un canal (38) pour transporter de l'eau pour le chauffage au travers du corps sont agencés dans le corps, **caractérisé en ce que** le pulvérisateur comprend un atomiseur pour la formation d'une pulvérisation de gouttelettes d'eau dans le lavabo, et dans lequel le dispositif de lavage comprend une alimentation en air qui est raccordée à l'atomiseur pour atomiser l'eau.

2. Dispositif de lavage selon la revendication 1, dans lequel le matériau thermoconducteur comprend un métal, de préférence de l'aluminium.

3. Dispositif de lavage selon la revendication 1 ou 2, dans lequel le canal présente un volume de moins de 400 ml, de préférence moins de 200 ml, de manière davantage préférée moins de 100 ml, et de manière préférée entre toutes un maximum de 50 ml.

4. Dispositif de lavage selon l'une quelconque des revendications précédentes, comprenant en outre un goutte-à-goutte.

5. Dispositif de lavage selon la revendication 4, dans lequel le goutte-à-goutte est configuré pour alimenter en eau à un débit de moins de 20 ml/s, de préférence moins de 10 ml/s, de manière davantage préférée moins de 5 ml/s et de manière préférée entre toutes un maximum de 2 ml/s.

6. Dispositif de lavage selon l'une quelconque des revendications précédentes, comprenant un conduit d'alimentation en air comprenant une pompe (18) et un filtre à air (28).

7. Dispositif de lavage selon la revendication 6, dans lequel le lavabo comprend en outre une ouverture d'extraction qui est raccordée via un conduit de recirculation d'air à la pompe pour la recirculation d'air.

8. Dispositif de lavage selon la revendication 6 ou 7, dans lequel le filtre à air (28) comprend une chambre à air sensiblement cylindrique (52), dans lequel une entrée d'air (56) est prévue dans la paroi latérale courbée de la chambre à air, laquelle entrée d'air est configurée pour former un flux circulaire dans la chambre à air, et dans lequel une sortie d'air (58) est prévue dans le côté supérieur ou inférieur de la chambre à air, laquelle sortie d'air présente un diamètre plus petit que le diamètre de la chambre à air et est prévue près de l'axe longitudinal de la chambre à air, dans lequel le filtre à air comprend une source de lumière UV (54) configurée pour faire briller la lumière UV dans la chambre à air.

9. Dispositif de lavage selon la revendication 8, dans lequel la paroi intérieure de la chambre à air (52) est dotée de dioxyde de titane.

10. Dispositif de lavage selon l'une quelconque des revendications 6 à 9, dans lequel la pompe est logée dans un boîtier thermo-isolant et est commutable au moyen d'un thermostat (50) entre un mode d'utilisation, dans lequel la pompe transporte de l'air via le conduit d'alimentation en air à l'atomiseur, et un mode de refroidissement dans lequel la pompe aspire de l'air extérieur.

11. Dispositif de lavage selon la revendication 10, dans lequel le corps de l'appareil de chauffage (34) est logé avec la pompe dans le boîtier thermo-isolant.

12. Dispositif de lavage selon l'une quelconque des revendications précédentes, dans lequel le pulvérisateur (10) comprend une ouverture de sortie, dans lequel un canal d'extraction raccordé à un extracteur est prévu tout autour de l'ouverture de sortie.

13. Dispositif de lavage selon l'une quelconque des revendications précédentes, comprenant en outre un dispositif de désionisation d'eau, de préférence un dispositif pour la désionisation capacitive d'eau.

14. Procédé de lavage d'objets, comprenant la fourniture d'un dispositif de lavage selon l'une quelconque des revendications 1 à 13.
